(19) Europäisches Patentamt / European Patent Office / Office européen des brevets

(11) **EP 3 925 594 A1**

(12) **EUROPEAN PATENT APPLICATION**
published in accordance with Art. 153(4) EPC

(43) Date of publication:
**22.12.2021 Bulletin 2021/51**

(51) Int Cl.:
*A61K 8/02* (2006.01)    *A61K 8/19* (2006.01)
*A61K 8/27* (2006.01)    *A61K 8/44* (2006.01)
*A61Q 19/02* (2006.01)

(21) Application number: **20773454.2**

(22) Date of filing: **10.03.2020**

(86) International application number:
**PCT/KR2020/003333**

(87) International publication number:
**WO 2020/189944 (24.09.2020 Gazette 2020/39)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **18.03.2019 KR 20190030701**

(71) Applicant: **Labnpeople Co.,Ltd.**
**Yangju-si, Gyeonggi-do 11477 (KR)**

(72) Inventor: **CHO, Sung Youn**
**Uijeongbu-si Gyeonggi-do 11812 (KR)**

(74) Representative: **Lahrtz, Fritz**
**Patentanwälte**
**Isenbruck Bösl Hörschler PartG mbB**
**Seckenheimer Landstr. 4**
**68163 Mannheim (DE)**

(54) **FLEXIBLE METAL PATCH HAVING ANTIOXIDANT ACTIVITY AND WHITENING EFFECT AND METHOD FOR USING SAME**

(57) The present invention relates to a flexible metal patch having antioxidant activity and brightening activity, the patch containing a biodegradable metal represented by Chemical Formula 1. The patch has DPPH free radical scavenge activity, thereby being useful in implementing antioxidant and brightening functions. Since the patch itself exhibits antioxidant activity and brightening activity without requiring combined use with other natural extracts or synthetic compounds, the patch can be easily used, can be easily locally applied to a region around or under the eyes. The patch is useful in preparation of cosmetics having effective antioxidant activity and brightening activity.

Chemical Formula 1        $Mg_aX_b$

where a and b are in units of % by weight, a+b = 100% by weight, 5 < a < 100, and X is any one or a combination of two or more selected from the group consisting of Zn, Ca, Fe, Mn, Si, Na, Zr, Ce, Ag, and P.

## Description

### Technical Field

The present invention relates to a flexible metal patch having antioxidant activity and brightening effect and, more particularly, to an antioxidant and brightening patch made of a biodegradable metal.

### Background Art

Skin brightening has been of constant interest as bright skin is regarded as a symbol of beauty. For this reason, the demand for skin brightening products is steadily increasing, and the cosmetic industry associated with such products is growing and has become a high value-added market.

It is no exaggeration to say that aging spots, freckles, rough skin, spots, and atopic dermatitis are skin abnormalities caused by an excess of active oxygen. Oxygen introduced into the body through respiration plays a role in preserving human life by generating energy required to sustain the body. Part of the oxygen that introduced into the body is converted into "active oxygen" through a chemical reaction, and the active oxygen is a beneficial substance that repels bacteria and serves as weapon used by blood cells. However, when these reactive oxygen species are excessively generated, they indiscriminately attack even our normal cells, killing or damaging them. Therefore, antioxidants that inhibit free radical reactions in vivo by reactive oxygen metabolites can be a kind of drug for preventing aging-related diseases. DPPH radical scavenging action is used as this antioxidant activity confirmation experiment.

There are various technologies relating to cosmetics having such 2,2-diphenyl-1-picrylhydrazyl (DPPH) free radical scavenging activity, antioxidant activity, and brightening activity, and such cosmetics are mostly based on various vegetable extracts (for example, Korean Patent Nos. 10-1811411, 10-1351187, and 10-1885224) or are based on a combination of specific compounds (for example, Korea Patent No. 10-1219520).

The inventors of the present patent application have conducted study on biocompatibility and strength retention of biodegradable Mg-Ca-Zn alloy bone graft materials (Assad M. 2012, "Biocompatibility and Strength Retention of Biodegradable Mg-Ca-Zn alloy Bone Implants", J Biomed Mater Res Part B 2012:00B:000-000, by Cho SY, Chae SW, Choi KW, Seok HK, Kim YC, Jung JY, Yang SJ, Kwon GJ, and Kim JT). Specifically, the biocompatibility and strength retention of a Mg-Ca-Zn alloy bone screw were evaluated. The results have revealed that in histopathological analysis using a rabbit implantation model, substantially no or minimal inflammatory cells appeared in the tissues around the Mg-Ca-Zn alloy implant. In addition, hematology and serum biochemical tests have proven to be within an acceptable range before and after transplantation, and it has been demonstrated that the release of metal atoms from the implant does not have any significant effect on normal tissues. In this respect, it has been confirmed that the Mg-Ca-Zn-based alloy is an excellent alternative to biodegradable polymers for orthopedics.

The inventors of the present patent application have confirmed the excellent biocompatibility of such a Mg-Ca-Zn-based alloy and have made attempt to use the Mg-Ca-Zn-based alloy in other fields. For example, the inventors have attempted to utilize the Mg-Ca-Zn-based alloy as a microcarrier. In this regard, the inventors of the present patent application have previously applied for a patent in connection with a microcarrier including Mg-based or Zn-based metals, for example, Mg alone, Zn alone, or Mg-Zn-Ca alloy (refer to Korean Patent Application Publication No. 2017-0115449). For these microcarriers, a human application test was conducted by the PNK Skin Clinical Research Center at the request of the inventors. Specifically, the efficacy and effectiveness of the microcarriers were evaluated by measuring the wrinkles around the eyes, skin tightness, dermal density, and skin thickness and checking the stability thereof.

### Disclosure

### Technical Problem

The objective of the present invention is to provide a flexible metal patch having antioxidant activity and brightening activity by using a biodegradable metal including a metal such as magnesium (Mg) or a metal alloy such as Mg alloy whose biocompatibility has been proven to be excellent through the inventors' research.

Specifically, the objective of the present invention is to provide a flexible metal patch having antioxidant activity and brightening functionality after confirming that the biodegradable metal itself expresses DPPH free radical scavenging activity.

### Technical Solution

In a first aspect of the present invention, there is provided a flexible metal patch having antioxidant activity and brightening activity, the metal patch containing a biodegradable metal represented by Chemical Formula 1.

[Chemical Formula 1]      $Mg_aX_b$

In Chemical Formula 1, a and b are weight% of each component, a+b = 100% by weight, 5<a<100, and X is one substance or a combination of two or more substances selected from the group consisting of Zn, Ca, Fe, Mn, Si, Na, Zr, Ce, Ag, and P.

In the flexible metal patch having antioxidant activity and brightening activity, according to one embodiment of the present invention, the biodegradable metal may satisfy a condition of $90 \leq a < 100$ in Chemical Formula 1.

In the flexible metal patch having antioxidant activity and brightening activity, according to one embodiment of the present invention, the biodegradable metal may be Mg with a purity of 95% or more.

In the flexible metal patch having antioxidant activity and brightening activity, according one an embodiment of the present invention, the biodegradable metal may be characterized in that two or more metal phases thereof may form a galvanic circuit to accelerate a decomposition rate.

In the flexible metal patch having antioxidant activity and brightening activity, according to one embodiment of the present invention, the biodegradable metal may include a $Mg_2Ca$ phase.

In the flexible metal patch having antioxidant activity and brightening activity, according to one embodiment of the present invention, the biodegradable metal may include a MgZn phase.

In the flexible metal patch having antioxidant activity and brightening activity, according to one embodiment of the present invention, the biodegradable metal may include a $Ca_2Mg_6Zn_3$ phase.

In the flexible metal patch having antioxidant activity and brightening activity, according to one embodiment of the present invention, the biodegradable metal may take a form in which a surface of a first metal is coated with a second metal different from the first metal, a polymer, or a ceramic material.

In the flexible metal patch having antioxidant activity and brightening activity, according to one embodiment of the present invention, the second metal in the biodegradable metal may be any one metal or a combination of two or more metals selected from the group consisting of sodium, magnesium, potassium, iron, nickel, zinc, gallium, selenium, strontium, zirconium, molybdenum, niobium, tantalum, titanium, silicon, silver, gold, manganese, and calcium.

The flexible metal patch having antioxidant activity and brightening activity, according to the preferred embodiment of the present invention, may be a patch including a microneedle.

In a second aspect of the present invention, there is provided a cosmetics package including: the flexible metal patch according to one of the embodiments of the first aspect, the flexible metal patch having antioxidant activity and brightening activity; and a cosmetic formulation including a compound having an effect of inhibiting tyrosinase activity and melamine production.

In the cosmetics package including the flexible metal patch having antioxidant and brightening activity, according to a preferred embodiment, the compound having the effect of inhibiting tyrosinase activity and melamine production may include tranexamic acid.

In a third aspect of the present invention, there is provided a method of using the flexible metal patch according to one of the embodiments of the first aspect, the method including a step of attaching the flexible metal patch to the skin.

The method of using the flexible metal patch according to a preferred embodiment may include a step of applying, to the skin, a cosmetic formulation including a compound having an effect of inhibiting tyrosinase activity or melamine production before or after attaching the flexible metal patch to the skin, in terms of obtaining a synergistic effect of antioxidant activity and brightening activity.

In this case, the compound having the effect of inhibiting tyrosinase activity or inhibiting melamine production may preferably include tranexamic acid.

## Advantageous Effects

The flexible metal patch containing a biodegradable metal or a biodegradable metal compound, according to the present invention, has DPPH free radical scavenging activity, thereby providing useful antioxidant and brightening functions. In addition, the flexible metal patch of the present invention is easy to use and is easy to locally apply to specific regions such as regions around and under the eyes because the flexible metal patch itself exhibits antioxidant activity and brightening activity without requiring combined use with extracts of other natural products or other chemicals. Therefore, it is possible to provide a multifunctional cosmetic formulation having antioxidant activity and brightening activity.

## Description of Drawings

FIG. 1 is a photograph of a specimen of a flexible metal patch made of 95% pure magnesium, in which the specimen is prepared for evaluation of antioxidant activity and brightening activity of the flexible metal patch according to the present invention.

FIG. 2 is a graph illustrating DPPH radical scavenging activity (%) according to the concentration of a methanol solution for a flexible metal patch according to the present invention, in which three curves correspond to respective wells.

FIG. 3 is a graph illustrating DPPH radical scavenging activity (%) according to the concentration of L-ascorbic acid in a methanol solution, the L-ascorbic acid being used as a positive control in the evaluation of DPPH radical scavenging activity of the flexible metal patch according to the present invention, in which three curves correspond to respective wells.

FIG. 4 is a graph illustrating improvement in skin brightness in clinical test results for a control group in which a patch and an ampoule are used in combination to evaluate the brightening function of the flexible metal patch according to the present invention.

FIG. 5 is a graph illustrating improvement in skin brightness in clinical test results for a test group in which a patch is used alone to evaluate the brightening function of the flexible metal patch according to the present invention.

**Best Mode**

Hereinafter, the present invention will be described in more detail with reference to the accompanying drawings.

As described above, the inventors of the present patent application have conducted research on the biocompatibility and effects for each application of magnesium or magnesium alloy and have reported the results in the form of a thesis or a patent document.

The present invention has been conceived as the result of continuous and extensive research and study. Through the research and study, the inventors have found that the biodegradable metal has DPPH free radical scavenging activity and have devised new uses of a flexible metal patch containing such a biodegradable metal in terms of antioxidant and brightening functions of the flexible metal patch.

In the specification of the present patent application, it will be demonstrated that a biodegradable metal itself has a DPPH radical scavenging ability, when acting alone.

In the examples of the present invention, a biodegradable metal using magnesium alone or an allow of magnesium and zinc or calcium was prepared in the form of a flexible metal patch, and the DPPH radical scavenging activity thereof was evaluated (Blois, MS 1958, "Antioxidant Determination by the Use of A Stable Free Radical", Nature 26, 1199-1200.). As a result, it was confirmed that a biodegradable metal prepared using magnesium alone as a first metal or an alloy of a first metal and a second metal has a specific range of DPPH radical scavenging activity.

Therefore, in a first aspect, the present invention provides an antioxidant and brightening flexible metal patch containing a biodegradable metal represented by Chemical Formula 1.

$$[Chemical\ Formula\ 1] \qquad Mg_aX_b$$

In Chemical Formula 1, a and b are weight% of each component, $a + b = 100\%$ by weight, $5 < a < 100$, and X is one substance or a combination of two or more substances selected from the group consisting of Zn, Ca, Fe, Mn, Si, Na, Zr, Ce, Ag, and P.

The biodegradable metal preferably includes at least 5% or more by weight of magnesium in terms of significantly expressing DPPH radical scavenging activity. In this regard, the biodegradable metal preferably satisfies a condition of $90 \leq a < 100$ in Chemical Formula 1.

The biodegradable metal according to the present invention is a metal having a property of being absorbed and decomposed to release metal ions and decomposition products in the body after being attached to the skin or inserted into the subcutaneous or epithelium. Magnesium (Mg), calcium (Ca), zinc (Zn) and the like are alkaline earth metal-based biodegradable metals. These metals have a mechanism of releasing hydrogen gas by reacting with water, as shown in Reaction Formulas 1 to 3 below.

$$[Reaction\ Formula\ 1] \qquad Mg + 2H_2O \rightarrow Mg(OH)_2 + H_2\ (gas)$$

$$[Reaction\ Formula\ 2] \qquad Ca + 2H_2O \rightarrow Ca(OH)_2 + H_2\ (gas)$$

$$[Reaction\ Formula\ 3] \qquad Zn + 2H_2O \rightarrow Zn(OH)_2 + H_2\ (gas)$$

In the present invention, it may be preferable that the biodegradable metal is composed of a single metal of magnesium (Mg) alone in terms of excellent biocompatibility and non-toxicity in normal cells or tissues. Here, the term "magnesium alone" means pure magnesium with a purity of 95% or more. That is, no other components are included in the pure magnesium, except for indispensable impurities generated in a metal production process.

On the other hand, the decomposition rate of the biodegradable metal represented by Chemical Formula 1 may be controlled in a manner that the biodegradable metal is composed of a metal alloy of two metals that differ in potential.

Specifically, the biodegradable metal may be a biodegradable metal including a $Mg_2Ca$ phase, a MgZn phase, or a $Ca_2Mg_6Zn_3$ phase.

Another method of controlling the decomposition rate of the biodegradable metal is that another metal (hereinafter, referred to as a second metal that is different from the metal serving as the main component of the biodegradable metal), a polymer, or a ceramic metal is coated on the surface of the biodegradable metal represented by Chemical Formula 1. In this case, examples of the second metal include sodium, magnesium, potassium, iron, nickel, zinc, gallium, selenium, strontium, zirconium, molybdenum, niobium, tantalum, titanium, silicon, silver, gold, manganese, calcium, and the like but are limited thereto. In this case, when the second metal is iron (Fe), chromium (Cr) and nickel (Ni), which are used to form stainless steel, must not be included. Examples of the polymer include poly lactic acid (PLA), poly glycolic acid (PGA), poly caprolactone (PCL), polydioxanone (PDO), and polymers thereof. Example of the ceramic material include magnesium oxide, magnesium hydroxide, hydroxylapatite, and beta-tricalcium phosphate (Beta-TCP).

As such, the biodegradable metal of the present invention has DPPH free radical scavenging activity. The inventors of the present patent application prepared a flexible metal patch made from a biodegradable metal that is composed of magnesium alone or a combination of magnesium and another metal such as zinc or calcium and evaluated their DPPH radical scavenging activity. The test result revealed that the biodegradable metal composed of magnesium alone or of an alloy in which another metal is mixed in a specific content range in addition to magnesium had DPPH radical scavenging activity.

In the description herein, the flexible metal patch is not limited in its shape. For example, the flexible metal patch may be a microneedle-type patch. One exemplary shape is disclosed in Korean Patent No. 10-1947624 (titled "Multi-type Microneedle") applied by and granted to the same inventors of the present patent application. Another exemplary shape is disclosed in Korean Patent Application Publication No. 10-2017-0115449 (titled "Microneedle Using Biodegradable Metal") applied by the same inventors of the present patent application and currently published.

In another aspect of the present invention, there is provided a cosmetics package for realizing the synergistic effect of antioxidant activity and brightening activity of the flexible metal patch having antioxidant activity and brightening activity as described above.

Specifically, the cosmetics package includes: the flexible metal patch according to one of the embodiments described above; a cosmetic formulation including a compound having an effect of inhibiting tyrosinase activity and inhibiting melamine production.

Melanin is a conjugate of black pigment and protein and is a component that determines human hair and skin color. When the melanin is present in a large amount, the skin color is in between yellowish brown and blackish brown. The smaller the amount, the lighter the skin color. When the melanin is present in a large amount, it is advantageous in terms of protecting the skin. However, it has the drawbacks of causing melasma, freckles, and skin spots. In addition, cell death or the progress of skin cancer may be promoted due to the toxicity of melanin precursors. Melanin is synthesized from melanocytes mainly observed in the basal layer of the epidermis or in the hair follicles below the basal layer, and the synthesized melanin is transported to keratinocytes through cytoplasmic protrusions to form epidermal melanin units. Melanin pigments generated from melanosomes in melanocytes move to adjacent keratinocytes through the dendrites of the melanosomes, and as the keratinocytes emerge as the epidermis, thereby appearing as skin color. Melanosomes contain specific enzymes necessary to synthesize normal melanin. The most well-known examples of the enzyme include tyrosinase, tyrosinase-related protein-1 (TRP-1), and tyrosinase-related protein-2 (TRP-2). Among them, tyrosinase is an enzyme that acts in the initial reaction, which is the rate-determining stage of melanin synthesis. Tyrosinase is a rate-limiting enzyme of melanin biosynthesis and biosynthesizes melanin by converting L-tyrosine to 3,4-dihydroxyphenylalanine (L-DOPA) and converting L-DOPA to DOPAquinone.

After this mechanism was reported, various compounds have been known to have an effect of inhibiting tyrosinase activity or inhibiting melamine production. The present invention provides a cosmetics package by combining a flexible metal patch that exhibits antioxidant activity and brightening activity by itself alone and a cosmetic formulation including a compound having the effect of inhibiting tyrosinase activity and inhibiting melamine production to increase a synergistic effect thereof.

Preferably, the compound having the effect of inhibiting tyrosinase activity and melamine production may include tranexamic acid, which is an antifibrinolytic agent and is known to have a therapeutic effect on age spots (blemishes), and post-inflammatory hyperpigmentation due to UV rays (UV-induced PIH). It is an organic acid and is used as an edible medicine as a blemish suppressant.

Melanin pigment, which is originally created to protect the skin, may cause age spots (blemishes) or pigmentation if it is excessively produced. When the tyrosinase enzyme is overstimulated, the melanin pigment is excessively secreted.

In this case, prostaglandin plays an important role in stimulating the tyrosinase. Tranexamic acid is a substance that inhibits the production of prostaglandins. Thus, the tranexamic acid is a compound known to be effective against age spots (blemishes).

Typically, tranexamic acid was developed as a drug to be eaten or applied after the treatment of melasma. In the case where tranexamic acid is prepared in the form of an oral drug, the absorption rate and reach rate are low. Therefore, it is provided as an application drug. However, since tranexamic acid is water-soluble, there has been a problem that it cannot be absorbed into the skin. However, when it is used in combination with the flexible metal patch having antioxidant activity and brightening activity provided in the present invention, the flexible metal patch not only expresses its own antioxidant and brightening activity but also effectively delivers tranexamic acid into the skin, resulting in increase in skin absorption of tranexamic acid. That is, the combined use provides a double effect.

Here, the cosmetic formulation including the compound (for example, tranexamic acid) having tyrosinase inhibitory activity or melamine production inhibitory activity is not limited in the formulation thereof and may be prepared in various known formulations. For example, it may be an ampoule formulation containing tranexamic acid but is not limited thereto.

The present invention also provides a method of using a flexible metal patch having antioxidant and brightening activity, the method comprising the step of attaching the flexible metal patch according to one of the embodiments to the skin.

The flexible metal patch according to the present invention is flexible due to the unique characteristics (modulus of elasticity of 40 to 45 GPa) of magnesium, so that the flexible metal patch can be easily attached conforming to the curved surface of the skin. Here, when the metal flexible patch is attached to the skin, the metal flexible patch itself can be attached alone, it may be attached via an adhesive material, or it may be attached by microneedles provided therein.

In consideration of the synergistic effect of antioxidant activity and brightening activity as described above, the step of applying a cosmetic formulation containing a compound having an effect of inhibiting tyrosinase activity and melamine production may be performed before or after the step of attaching the flexible metal patch.

Specifically, it is possible to provide a usage method that can achieve the effect of promoting skin absorption by applying a cosmetic formulation containing tranexamic acid having a low absorption rate when applied to the skin.

**Mode for Invention**

Hereinafter, the present invention will be described in detail with respect to Examples, but the present invention is not limited by the Examples.

**<Example 1>**

To evaluate the DPPH free radical scavenging activity of the biodegradable metal of the present invention, samples were provided to the Korea Testing & Research Institute and the test thereof was performed in a manner described below by the Korea Testing & Research Institute at the request of the inventors.

(1) Materials

a. Test substance

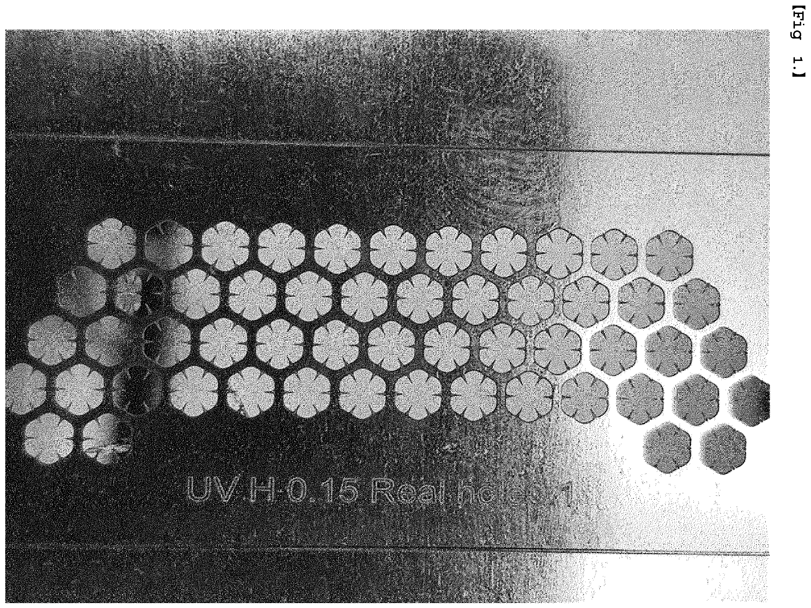

- Magnesium patch with a purity of 95% or more (See FIG. 1 in which the patch is provided with microneedles that are not bent).
- The sample used for the test is a sheet shape (dimensions: 2.6 mm $\times$ 2.4 mm, thickness 0.1 t) made of Mg with a purity of 95% or more and including unavoidable impurities generated during manufacturing thereof.

b. Positive control

- L-ascorbic acid: Sigma-Aldrich Corporation, Lot No. SLBR2586V

c. Etc.

- 2,2-Diphenyl-1-picrylhydrazyl (DPPH): Sigma-Aldrich Corporation, Lot No. STBH0044
- Methanol: JINSEI, Lot No. 2016G2012

(2) Test method

a. Preparation of test substance:

The provided test substance was used without conversion of purity. Methanol was used to adjust the concentration.

b. Preparation of positive control substance

After weighing L-ascorbic acid, it was prepared to have a predetermined concentration using methanol.

c. Analysis of the test substance:

In consultation with the client, the analysis on the stability and homogeneity of the test substance was not conducted.

d. DPPH preparation

DPPH was prepared to have a concentration of 0.2 mM using methanol.

e. Test group composition

A test group was composed as shown in Table 1 below.

[Table 1]

| Test group | Test substance | Treatment concentration (%) |
|---|---|---|
| G1(test substance) | Magnesium patch | 1, 2.5, 5, 10 |
| G2(positive control subs tance) | L-ascorbic acid | 0.0005, 0.001, 0.0015, 0.002 |

f. Substance application

In a 96-well plate, each of the preparations (test substance and positive control substance) having different concentrations was mixed with a combination of 0.2 mM DPPH and methanol in a mixing ratio of 1:1.

g. Sample solution reaction and absorbance measurement

After reacting at room temperature for 30 minutes in a light-shielding state, centrifugation was performed to obtain a supernatant, and absorbance was measured at a wavelength of 517 nm.

h. Results

The free radical scavenger activity (%) of DPPH was calculated using Equation 1 below.

<Equation 1>

DPPH radical scavenging activity(%) = 1-(A/B)×100

In Equation 1, A is the absorbance of the test substance, and B is the absorbance of the blank sample.

i. Statistical processing

The result of each test was measured three times to obtain the mean value and standard deviation, and statistical analysis was performed using the SPSS statistical program (Ver. 19.0). First, after performing Levene's test, a one-way ANOVA test was performed. When significance was confirmed among the test groups, a post-hoc test was performed depending on the presence or absence of homogeneity of variances (when the variances are homogeneous, Duncan's multiple test was performed whereas when the variances were heterogeneous, Dunnett's T3 was performed).

(3) Result

a. DPPH free radical scavenging activity (%)

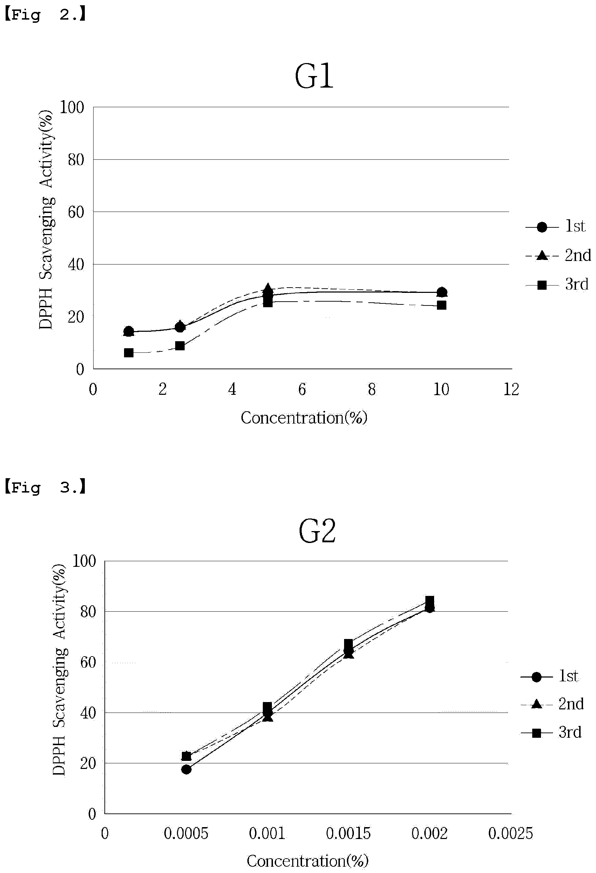

As shown in Table 2 below and in FIGS. 2 and 3, the test group G1 using a magnesium patch as a test substance was prepared in concentrations of 1%, 2.5%, 5%, and 10% to measure DPPH free radical scavenge activity. As a result, the activity was 11.58%, 13.73%, 27.69%, and 27.55% at the respective prepared concentrations of the test substance.

[Table 2]

| Test group | Number of wells | Concentrati on (%) | $OD_{517}$(mean $\pm$ standard deviation) | DPPH free radical scavenge activity (%) (mean $\pm$ standard deviation) |
|---|---|---|---|---|
| G1 | 3 | 0 | 0.859 $\pm$ 0.041 | - |
| | 3 | 1 | 0.758 $\pm$ 0.017 | 11.58 $\pm$ 4.69 |
| | 3 | 2.5 | 0.740 $\pm$ 0.014 | 13.73 $\pm$ 4.22 |
| | 3 | 5 | 0.620 $\pm$ 0.008 | 27.69 $\pm$ 2.57 |
| | 3 | 10 | 0.622 $\pm$ 0.017 | 27.55 $\pm$ 3.06 |
| G2 | 3 | 0 | 0.610 $\pm$ 0.029 | - |
| | 3 | 0.0005 | 0.482 $\pm$ 0.024 | 20.96 $\pm$ 2.79 |
| | 3 | 0.001 | 0.364 $\pm$ 0.027 | 40.38 $\pm$ 1.61 |
| | 3 | 0.0015 | 0.210 $\pm$ 0.023 | 65.57 $\pm$ 2.21 |
| | 3 | 0.002 | 0.102 $\pm$ 0.013 | 83.24 $\pm$ 1.48 |

As described above, to evaluate the antioxidant effect of the magnesium patch, a free radical scavenging activity test using the 2,2-Diphenyl-1-picrylhydrazyl (DPPH) method was conducted. In the test, the magnesium patch as a test substance was prepared as solutions having concentrations of 1%, 2.5%, 5% and 10%, the scavenging activity was 11.58%, 13.73%, 27.69%, and 27.55% at the respective concentrations. In the case of the positive control substance, the antioxidant activity was measured to be 20.96%, 40.38%, 65.57%, and 83.24% at concentrations of 0.0005%, 0.001%, 0.0015%, and 0.002%, respectively, and the $IC_{50}$ (which means a concentration at which 50% activity is exhibited) was 0.0012%.

The above results reveal that in the test for measuring DPPH free radical scavenging activity, the magnesium patch, which is the test substance, seems to have an antioxidant effect at a concentration of 5% or more.

**<Example 2>**

To evaluate the brightening function of the biodegradable metal of the present invention, clinical evaluation was performed in a manner described below by P&K Skin Research Center at the request of the inventors.

(1) Test method

1) Evaluation items

a. Instrumental evaluation - skin brightness measurement (CM-700d, Konica Minolta, Japan)
b. Global Assessment of Efficacy - Subject Assessment
c. Safety evaluation
At each visit, the researcher evaluated the effects by combining the researcher's visual investigation of the site where the test product was applied and the results of the subject's questionnaire.
d. Other investigation (observation) items.

- Demographic survey: survey on gender, date of birth, and age before starting the human application test.
- Investigation of vital signs: physical examination through visual observation before the start of the human application test.
- Medical history survey: investigation of main symptoms, onset date of the symptoms, diagnosis and treatment history for the symptoms before starting the human application test of the biodegradable metal of the present invention.
- Product preference survey: investigation of product preference through questionnaire data

2) Visit schedule: 5 visits

a. First visit: selection of test subjects, the consent of the subjects, measurement before use, delivery of test products
b. Second visit: measurement of skin and adverse reaction evaluation after 2 weeks of use
c. Third visit: measurement of skin and adverse reaction evaluation after 4 weeks of use
d. Fourth visit: measurement of skin and adverse reaction evaluation after 4 weeks of use, and test product recall
e. Fifth visit: after 2 weeks of stop of use, skin measurement, adverse reaction evaluation, efficacy survey, and preference survey were performed.

3) Primary efficacy endpoints: measurement values representing improvement in skin brightness under the eyes before use of the test product, after 2 weeks of use, after 4 weeks of use, after 8 weeks of use, and 2 weeks after the stop of use of the test product.
4) Secondary efficacy endpoints: results of global assessment of efficacy
5) Safety evaluation: evaluation of abnormal symptoms that appear after using the test product.

(2) Test results

This test was conducted on women aged 29 to 55 years. The women used the "magnesium patch as shown in FIG. 1 (specifically, a magnesium microneedle patch with bent microneedles)" for eight weeks and then did not used the magnesium patch for two weeks thereafter. After that, the improvement in skin brightness in the area under the eyes was measured. The magnesium microcarrier multi-care patch (tentative name for the product of the present invention) and ampoules were used in combination on the left side of the face, and the magnesium microcarrier multi-care patch was used alone on the right side of the face. After the end of the test, the survey on the efficacy and preference was performed.

1) Subjects who have completed the test
All 22 subjects (except for 2 dropouts) were female and the average age thereof was 45.23 years. The selected subjects did not have nay specific skin symptoms and had no history of diseases or medications that could affect the study.
2) When checking the skin brightness after using the test product, the skin brightness on the left side of the face, on which the magnesium microneedle patch and the ampoule were used in combination, was increased significantly (p < 0.05) after 8 weeks of use compared to the state before the use of the product. There was no significant difference (p < 0.05) between the facial state after 2 weeks of the stop of the use of the product and the facial state after 8 weeks of use of the product. On the other hand, in the case of the right side of the face, on which the magnesium microneedle patch alone was used, the skin brightness increased significantly (p < 0.05) after 4 weeks of use and after 8 weeks of use compared to the state before using the product. There was no significant difference (p < 0.05) between the state after 2 weeks of the stop of use of the product and the state immediately after 8 weeks of product use.

The detailed evaluation results are shown in Table 3 below. The results in Table 3 below are expressed in the form of "standard ± standard deviation" values.

[Table 3]

| Classification | Combined use of patch and ampoule | Patch only |
| --- | --- | --- |
| Before use | 58.755 ± 2.190 | 58.955 ± 2.196 |

(continued)

| Classification | Combined use of patch and ampoule | Patch only |
|---|---|---|
| After two weeks of use | 59.043 ± 2.287 | 59.494 ± 2.218 |
| After four weeks of use | 59.082 ± 2.133 | 59.460 ± 2.013 |
| After eight weeks of use | 59.573 ± 2.278 | 59.551 ± 2.091 |
| Two weeks after stop of use | 59.397 ± 1.920 | 59.378 ± 1.948 |

For the above results, the in-group comparison significance probability (p-value) was calculated, and the results are shown in Table 4 below.

[Table 4]

| Classification | 0.001* | 0.001* |
|---|---|---|
| After two weeks of use - before use | 1.000 | 0.053 |
| After four weeks of use - before use | 1.000 | 0.013* |
| After eight weeks of use - before use | 0.001* | 0.006* |
| Two weeks after stop of use - after eight weeks of use | 1.000 | 0.516 |
| (Note) *: p <0.05 by repeated measures ANOVA, post hoc Bonferroni correction | | |

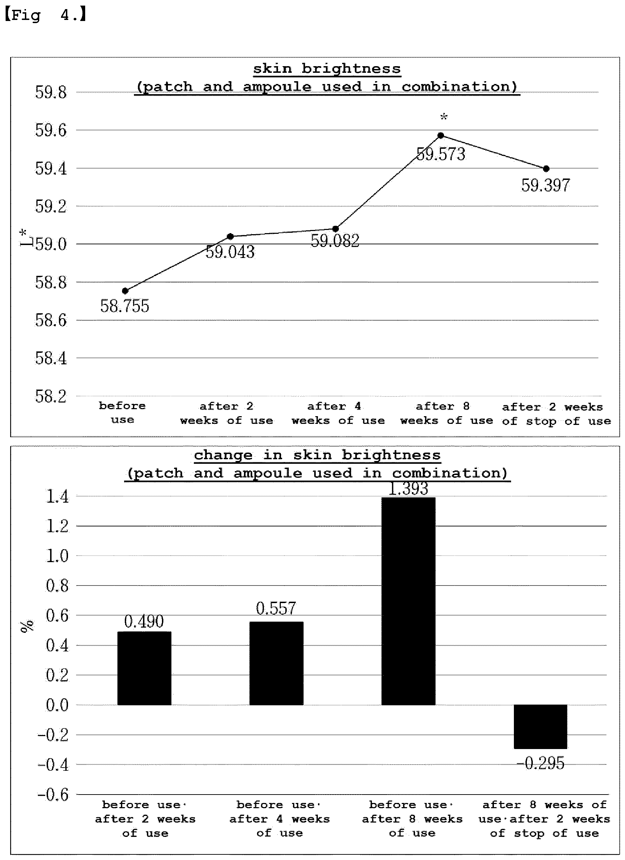

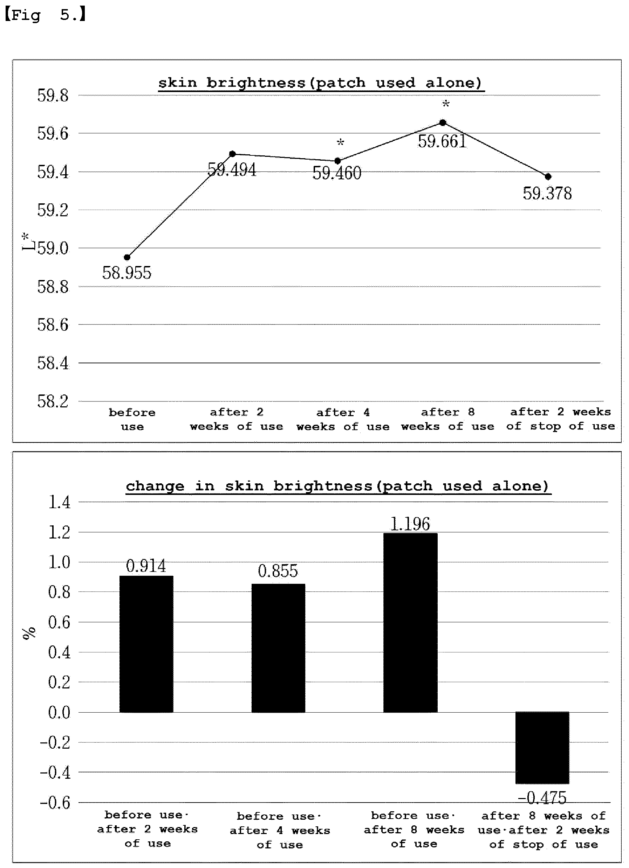

The above results are graphically shown in FIGS. 4 and 5, respectively.

On the other hand, the result of the effectiveness evaluation questionnaire for the test product has revealed that 100.00% of the test subjects rated the skin brightness improvement as above average. In addition, there were no reports of any special skin adverse reactions during the period in which the subjects used the test product, and no abnormal findings were observed in the physical examination by a dermatologist.

From the above results, it was confirmed that the magnesium microneedle patch alone as an embodiment of the present invention improved skin brightness when it was used for 8 weeks in a manner to be locally applied to the area under the eye on the right side of the face and maintained the improved skin brightness for two weeks after 8 weeks of product use.

**Industrial Applicability**

The flexible metal patch containing a biodegradable metal or metal compound, according the present invention, is useful as an antioxidant and brightening material. Since the flexible metal patch itself exhibits antioxidant activity and brightening activity without requiring combined use with an extract of a natural product or with a synthetic compound, the flexible metal patch is easy to use and is easy to locally apply to an area around or under the eyes. Therefore, it is useful in preparing a multifunctional cosmetic formation having antioxidant and brightening functions.

**Claims**

1. A flexible metal patch having antioxidant and brightening activity, the flexible metal patch comprising a biodegradable metal represented by Chemical Formula 1,

  [Chemical Formula 1]    $Mg_aX_b$

  wherein in Chemical Formula 1, a and b are weight% of each component, a + b = 100% by weight, 5 < a < 100, and X is any one or a combination of two or more selected from the group consisting of Zn, Ca, Fe, Mn, Si, Na, Zr, Ce, Ag, and P.

2. The flexible metal patch according to claim 1, wherein the biodegradable metal satisfies a condition of $90 \leq a < 100$ in Chemical Formula 1.

3. The flexible metal patch according to claim 1, wherein the biodegradable metal is Mg having a purity of 95% or more.

4. The flexible metal patch according to claim 1, wherein the biodegradable metal has two or more metal phases that form a galvanic circuit so that the decomposition rate thereof is increased.

5. The flexible metal patch according to claim 4, wherein the biodegradable metal comprises an $Mg_2Ca$ phase.

6. The flexible metal patch according to claim 4, wherein the biodegradable metal comprises a MgZn phase.

7. The flexible metal patch according to claim 4, wherein the biodegradable metal comprises a $Ca_2Mg_6Zn_3$ phase.

8. The flexible metal patch according to claim 1, wherein the biodegradable metal is coated with a second metal that is different from the biodegradable metal, a polymer, or a ceramic material.

9. The flexible metal patch according to claim 8, wherein the second metal is any one or a combination of two or more selected from the group consisting of sodium, magnesium, potassium, iron, nickel, zinc, gallium, selenium, strontium, zirconium, molybdenum, niobium, tantalum, titanium, silicon, silver, gold, manganese, and calcium.

10. The flexible metal patch according to any one of claims 1 to 9, wherein the flexible metal patch is a patch comprising microneedles.

11. A cosmetics package comprising:

   the flexible metal patch according to claim 1; and
   a cosmetic formulation comprising a compound having an effect of inhibiting tyrosinase activity to inhibiting melamine production.

12. The cosmetics package according to claim 11, wherein the compound having the effect of inhibiting tyrosinase activity or inhibiting melamine production comprises tranexamic acid.

13. A method of using the flexible metal patch according to claim 1, the method comprising a step attaching the flexible metal patch of claim 1 to the skin.

14. The method according to claim 13, further comprising a step of applying a chemical formulation comprising a compound having an effect of inhibiting tyrosinase activity and melamine production, the applying being performed before or after the attaching of the flexible metal patch.

15. The method according to claim 14, wherein the compound having the effect of inhibiting tyrosinase activity and melamine production comprises tranexamic acid.

【Fig 1.】

【Fig 2.】

## G1

【Fig 3.】

## G2

【Fig 4.】

skin brightness
(patch and ampoule used in combination)

change in skin brightness
(patch and ampoule used in combination)

【Fig 5.】

skin brightness(patch used alone)

58.955 · 59.494 · 59.460 · 59.661* · 59.378

before use · after 2 weeks of use · after 4 weeks of use · after 8 weeks of use · after 2 weeks of stop of use

change in skin brightness(patch used alone)

0.914 · 0.855 · 1.196 · −0.475

before use· after 2 weeks of use · before use· after 4 weeks of use · before use· after 8 weeks of use · after 8 weeks of use·after 2 weeks of stop of use

## INTERNATIONAL SEARCH REPORT

| | International application No. |
|---|---|
| | **PCT/KR2020/003333** |

**A. CLASSIFICATION OF SUBJECT MATTER**

*A61K 8/02(2006.01)i, A61K 8/19(2006.01)i, A61K 8/27(2006.01)i, A61K 8/44(2006.01)i, A61Q 19/02(2006.01)i*

According to International Patent Classification (IPC) or to both national classification and IPC

**B. FIELDS SEARCHED**

Minimum documentation searched (classification system followed by classification symbols)

A61K 8/02; A23L 1/30; A61K 31/409; A61K 33/06; A61K 47/02; A61L 27/54; A61L 31/10; A61M 37/00; C07K 19/00; A61K 8/19; A61K 8/27; A61K 8/44; A61Q 19/02

Documentation searched other than minimum documentation to the extent that such documents are included in the fields searched
Korean utility models and applications for utility models: IPC as above
Japanese utility models and applications for utility models: IPC as above

Electronic data base consulted during the international search (name of data base and, where practicable, search terms used)
eKOMPASS (KIPO internal) & Keywords: tranexamic acid, whitening, anti-oxidant, magnesium (Mg), metal

**C. DOCUMENTS CONSIDERED TO BE RELEVANT**

| Category* | Citation of document, with indication, where appropriate, of the relevant passages | Relevant to claim No. |
|---|---|---|
| X | KR 10-2017-0115449 A (LABNPEOPLE CO., LTD.) 17 October 2017<br>See abstract; paragraphs [0031], [0043], [0046]; table 1; claims 1-13. | 1-12 |
| A | KR 10-2011-0065391 A (U&I CORPORATION) 15 June 2011<br>See abstract; claims 1-8. | 1-12 |
| A | KR 10-1352565 B1 (SOGANG UNIVERSITY RESEARCH & BUSINESS DEVELOPMENT FOUNDATION) 17 January 2014<br>See abstract; paragraphs [0002]-[0004]; figure 1; claim 1. | 1-12 |
| A | KR 10-1483207 B1 (KOREA INSTITUTE OF GEOSCIENCE AND MINERAL RESOURCES(KIGAM)) 22 January 2015<br>See paragraph [0067]; claim 1. | 1-12 |
| A | TSE, T. W. et al. Tranexamic acid: an important adjuvant in the treatment of melasma. Journal of cosmetic dermatology. 2013, vol. 12, pages 57-66<br>See abstract; page 58. | 1-12 |

☐ Further documents are listed in the continuation of Box C.   ☒ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 12 JUNE 2020 (12.06.2020) | **15 JUNE 2020 (15.06.2020)** |

| Name and mailing address of the ISA/KR | Authorized officer |
|---|---|
| Korean Intellectual Property Office<br>Government Complex Daejeon Building 4, 189, Cheongsa-ro, Seo-gu, Daejeon, 35208, Republic of Korea<br>Facsimile No. +82-42-481-8578 | |
| | Telephone No. |

Form PCT/ISA/210 (second sheet) (January 2015)

## INTERNATIONAL SEARCH REPORT

| International application No. |
|---|
| PCT/KR2020/003333 |

---

**Box No. II     Observations where certain claims were found unsearchable (Continuation of item 2 of first sheet)**

This international search report has not been established in respect of certain claims under Article 17(2)(a) for the following reasons:

1. ☒ Claims Nos.: **13-15**
   because they relate to subject matter not required to be searched by this Authority, namely:

   Claims 13-15 include a method invention which includes a step for direct treatment of the human body and which can provide a treatment effect, and thus pertain to a subject matter on which the International Searching Authority is not required to carry out an international search under the provisions of PCT Article 17(2)(a)(i) and PCT Rule 39.1(iv).

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

---

**Box No. III     Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**     ☐ The additional search fees were accompanied by the applicant's protest and, where applicable, the payment of a protest fee.
☐ The additional search fees were accompanied by the applicant's protest but the applicable protest fee was not paid within the time limit specified in the invitation.
☐ No protest accompanied the payment of additional search fees.

Form PCT/ISA/210 (continuation of first sheet (2)) (January 2015)

| | | International application No. |
|---|---|---|
| | | **PCT/KR2020/003333** |

| Patent document cited in search report | Publication date | Patent family member | Publication date |
|---|---|---|---|
| KR 10-2017-0115449 A | 17/10/2017 | BR 112018070658 A2 | 05/02/2019 |
| | | BR 112018070666 A2 | 05/02/2019 |
| | | CN 109069812 A | 21/12/2018 |
| | | CN 109069814 A | 21/12/2018 |
| | | EP 3441107 A2 | 13/02/2019 |
| | | EP 3444003 A1 | 20/02/2019 |
| | | JP 2019-510618 A | 18/04/2019 |
| | | JP 2019-515949 A | 13/06/2019 |
| | | KR 10-1879851 B1 | 18/07/2018 |
| | | KR 10-2017-0115429 A | 17/10/2017 |
| | | KR 10-2017-0115431 A | 17/10/2017 |
| | | KR 10-2017-0115458 A | 17/10/2017 |
| | | KR 10-2019-0075033 A | 28/06/2019 |
| | | KR 10-2074388 B1 | 06/02/2020 |
| | | KR 10-2114984 B1 | 26/05/2020 |
| | | MX 2018012236 A | 28/03/2019 |
| | | MX 2018012334 A | 28/03/2019 |
| | | US 2019-0091456 A1 | 28/03/2019 |
| | | US 2019-0151638 A1 | 23/05/2019 |
| | | WO 2017-176069 A2 | 12/10/2017 |
| | | WO 2017-176069 A3 | 30/11/2017 |
| | | WO 2017-176077 A1 | 12/10/2017 |
| KR 10-2011-0065391 A | 15/06/2011 | None | |
| KR 10-1352565 B1 | 17/01/2014 | KR 10-2013-0087926 A | 07/08/2013 |
| KR 10-1483207 B1 | 22/01/2015 | None | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 101811411
- KR 101351187
- KR 101885224
- KR 101219520

- KR 20170115449
- KR 101947624
- KR 1020170115449

**Non-patent literature cited in the description**

- **ASSAD M. ; CHO SY ; CHAE SW ; CHOI KW ; SE-OK HK ; KIM YC ; JUNG JY ; YANG SJ ; KWON GJ ; KIM JT.** Biocompatibility and Strength Retention of Biodegradable Mg-Ca-Zn alloy Bone Implants. *J Biomed Mater Res Part B,* 2012, vol. 2012 (00B), 000-000

- **BLOIS, MS.** Antioxidant Determination by the Use of A Stable Free Radical. *Nature,* 1958, vol. 26, 1199-1200